# EUROPEAN PATENT SPECIFICATION

(11) **EP 2 295 975 B1**
(45) Date of publication and mention of the grant of the patent: **18.06.2014**
(21) Application number: 10175852.2
(22) Date of filing: 08.09.2010
(51) Int. Cl.: G01N 33/574

(54) **Determining the expression status of human epidermal growth factor receptor 2 (HER2) in biological samples**
Bestimmung des Expressionsstatus des humanen epidermischen Wachstumsfaktorrezeptors 2 (HER2) in biologischen Proben
Détermination du statut d'expression du récepteur du facteur de croissance épidermique humain 2 (HER2) dans des échantillons biologiques

(30) Priority: 10.09.2009 US 241253 P
(43) Date of publication of application: 16.03.2011
(60) Divisional application: 13182442.7
(73) Proprietor: Bruker Daltonik GmbH, 28359 Bremen (DE)
(72) Inventor: Albers, Christian, 28357 Bremen (DE); Belau, Eckhard, 28865 Lilienthal (DE); Deininger, Sören-Oliver, 04277 Leipzig (DE); Rauser, Sandra, 81675 München (DE); Suckau, Detlef, 28879 Grasberg (DE); Walch, Axel, 85598 Baldham (DE)
(74) Representative: Kohler Schmid Möbus

(56) References cited:
- US-A1- 2008 176 258
- ZHANG D ET AL: "Proteomic study reveals that proteins involved in metabolic and detoxification pathways are highly expressed in HER-2/neu-positive breast cancer", MOLECULAR AND CELLULAR PROTEOMICS 200511 US LNKD- DOI:10.1074/MCP.M400221-MCP200, vol. 4, no. 11, November 2005 (2005-11), pages 1686-1696, XP002602356, ISSN: 1535-9476
- JUSTENHOVEN C ET AL: "Polymorphic loci of E2F2, CCND1 and CCND3 are associated with HER2 status of breast tumors", INTERNATIONAL JOURNAL OF CANCER 20090501 WILEY-LISS INC. USA LNKD- DOI:10.1002/IJC.24198, vol. 124, no. 9, 1 May 2009 (2009-05-01), pages 2077-2081, XP002602357,
- MACKAY A ET AL: "CDNA MICROARRAY ANALYSIS OF GENES ASSOCIATED WITH ERBB2 (HER2/NEU) OVEREXPRESSION IN HUMAN MAMMARY LUMINAL EPITHELIAL CELLS", ONCOGENE, NATURE PUBLISHING GROUP, GB, vol. 22, no. 17, 1 May 2003 (2003-05-01), pages 2680-2688, XP009018980, ISSN: 0950-9232, DOI: DOI:10.1038/SJ.ONC.1206349
- HAO JIHUA ET AL: "Identification and rational redesign of peptide ligands to CRIP1, a novel biomarker for cancers.", PLOS COMPUTATIONAL BIOLOGY 2008 LNKD- PUBMED:18670594, vol. 4, no. 8, 2008, page E1000138, XP002615159, ISSN: 1553-7358
- MA X -J ET AL: "Gene expression profiles of human breast cancer progression", PROCEEDINGS OF THE NATIONAL ACADEMY OF SCIENCES OF THE UNITED STATES OF AMERICA 20030513 US LNKD- DOI:10.1073/PNAS.0931261100, vol. 100, no. 10, 13 May 2003 (2003-05-13) , pages 5974-5979, XP002615160, ISSN: 0027-8424
- LIU SHUQIAN ET AL: "Thiamine transporter gene expression and exogenous thiamine modulate the expression of genes involved in drug and prostaglandin metabolism in breast cancer cells.", MOLECULAR CANCER RESEARCH : MCR AUG 2004 LNKD- PUBMED:15328374, vol. 2, no. 8, August 2004 (2004-08), pages 477-487, XP002615161, ISSN: 1541-7786

## Description

### Background of the Invention

Adenocarcinoma of the breast is a leading cause of cancer morbidity and mortality among women worldwide. A major challenge faced by clinicians treating patients with breast cancer is how to best assess patient outcome and predict the clinical course of the disease so that the most appropriate treatment regimen can be identified. Determining the expression status of the human epidermal growth factor receptor 2 (HER2) in newly diagnosed breast cancer tissues is critically important for therapeutic decision making. Current guidelines recommend that the HER2 expression status should be evaluated for every patient with newly diagnosed, primary breast cancer. Unlike most histopathologic testing, which serves as an adjunct to establishing a diagnosis, the results of HER2 testing stand alone in determining which patients afflicted with primary breast cancer are likely to respond to trastuzumab (Herceptin^{®}), a monoclonal antibody directed to HER2. The HER2 expression status can be determined at the DNA-, the mRNA-, or the protein level. Various techniques are available for assessing these different target molecules, each with benefits and drawbacks. Although almost a decade has passed since trastuzumab was initially approved by the USA Food and Drug Administration (FDA), accurate HER2 expression status determination continues to challenge the field of clinical laboratory testing. Currently, two testing methods are approved by the FDA for HER2 testing of breast cancer tissues in the laboratory: immunohistochemical analysis (IHC) and fluorescence in situ hybridization (FISH).

There are previous findings using gene transcription profiles that show an increased transcription of the gene related to the human cystein-rich intestinal protein 1 (CRIP1) in human breast cancers compared to non-diseased tissues (Ma et al., 2003; Liu et al., 2004) and other cancer types. In experiments comparing transcription of the CRIP1 gene in human breast cancer to matched normal breast tissue, the mRNA for this target was over-expressed 8 to 10-fold (Ma et al., 2003). Two recent studies compared differential gene transcription patterns in HER2-positive and HER2-negative breast cancer cell lines and tissues (Wilson et al., 2002; Mackay et al., 2003), wherein the mRNA of the CRIP1 gene and HER2-positive were found to be both up-regulated. Justenhoven et al. studied transcriptional regulation and analyzed 3 polymorphisms of genes associated with HER2 status of breast tumors (Int. Journal of Cancer, 2009, 124(9), 2077-2081: "Polymorphic loci of E2F2, CCND1 and CCND3 are associated with HER2 status of breast tumors"). In contrast to these genetic findings, an increased expression of the CRIP1 at the protein level has not been shown yet. Zhang et al. found nine proteins to be up-regulated in HER2 positive breast tumors (Molecular and cellular Proteomics, 2005, 4(11), 1686-1696: "Proteomic study reveals that proteins involved in metabolic and detoxification pathways are highly expressed in HER-2/neu-positive breast cancer"). In their proteomic study, to identify the proteins that are associated with the aggressive phenotype of HER2 positive breast cancer, tumor cells from both HER2 positive and -negative tumors were procured by laser capture micro dissection. Differentially expressed proteins in the two subsets of tumors were identified by two-dimensional electrophoresis and MALDI-TOFKOF MS/MS. Pastorek et al. showed the coexpression of CA IX (carbonic anhydrase IX) and HER-2/neu/c-erbB-2 ("HER -2"), and diagnostic/prognostic and therapeutic methods in parallel with and/or alternative to those targeting HER-2. Particularly preferred were assays to detect both the HER-2 ectodomain ("p100") and the CA IX extracellular domain (50/54 kilodaltons) in the same body fluid sample from a cancer patient.

CRIP1 belongs to the LIM/double zinc finger protein family, which includes cysteine- and glycine-rich protein-1, rhombotin-1, rhombotin-2, and rhombotin-3. Human CRIP1, primarily a cytosolic protein, was cloned in 1997 using RT-PCR of human small intestine RNA and oligonucleotides whose sequence was derived from the human heart homologue of this protein, CRHP.

Mass spectrometry has extensively been used for investigating protein patterns in biological samples, like body fluids or homogenized tissue, to find biomarkers. Interrogation of the resulting complex mass spectrometry (MS) data sets using modem computational tools has resulted in identification of both disease-state and patient-prognosis specific protein patterns. In general, biomarkers are used or at least predicted to be used for diagnosing cancer, qualifying different types of cancer, predicting the response to a cancer drug or predicting the prognosis of a cancer patient. Biomarkers can further be measured and optionally be identified with high accuracy by tandem mass spectrometry, e.g. by multiple reaction monitoring (MRM).

Matrix-assisted laser desorption/ionization (MALDI) imaging mass spectrometry (IMS) is a powerful tool for investigating protein patterns through the direct (in situ) analysis of tissue sections. Similarly to IHC and FISH, MALDI IMS has advantage over other assay methods (i.e. those requiring homogenization), because it is morphologically driven. This allows the direct evaluation of tumor cells, correlation with other morphologic features and the ability to assay smaller patient tumor tissue specimen such as needle core biopsy specimens. This makes it a seemingly ideal tool for rapid tissue diagnostics and molecular histology. Besides, MALDI IMS can determine the distribution of hundreds of compounds in a single measurement without any need of labeling.

Previously published work using MALDI IMS for tumor classification suggests that this technology enables accurate classification (Yanagisawa et al., 2003; Schwartz et al., 2005). To date, MALDI IMS has been applied to multiple types of diseased tissues, including human non-small cell lung tumors, gliomas, as well as ovarian and breast cancers. From recent publications it has become clear that the integration of MALDI IMS into clinical management regarding disease diagnosis and outcome prediction will take place soon (Franck et al., 2009; Cornett et al., 2007). As MALDI IMS protein expression data will become available for various tumor tissue types, this approach will provide a common disease-wide methodology that can be applied to a variety of clinical questions. While many of these MALDI IMS studies rather focus on the identification of new biomarkers, so far only few studies have evaluated the potential of MALDI IMS for molecular classification of tissues based on protein patterns.

### Summary of the Invention

The present invention provides a method as defined in claim 1.

Preferably, the amount of at least one further protein is measured and compared to a control amount, the at least one further protein having a molecular weight of 4740, 8455, 8570, 8607 or 8626 atomic mass units and being related to fragments of the human epidermal growth factor receptor 2. Preferably, the amount of at least one further protein is measured and compared to a control amount, the at least one further protein having a molecular weight of 8435, 8450 or 8465 atomic mass units and being related to variants of the human cystein-rich intestinal protein 1. Preferably, three or more of the marker proteins are measured in step (b). Most preferably, all seven marker proteins are measured in step (a). The proteins of molecular weights of 4740, 8455, 8570, 8607 and 8626 atomic mass units are presumably related to fragments of higher molecular weight proteins correlated with HER2, whereas the marker protein of molecular weight of 8404 and 8419 atomic mass units are related within normal mass accuracy to the human cystein-rich intestinal protein 1 (CRIP1) and a variant thereof, respectively. The underivatized CRIP1_HUMAN des-Met 1 has an average molecular weight of 8402 u calculated based on the gene sequence. The modified molecular weight of the variant can be a result of a modification, a post-translational modification, a sequence variation, a point mutation and/or a sequence length variation. The variants of the human cystein-rich intestinal protein 1 (CRIP1 variants) have preferably one of following molecular weights 8419, 8435, 8450 or 8465 atomic mass units. These and other variations can result from one or more structural variations, wherein the human cystein-rich intestinal protein 1 is modified, e.g., by disulfide bond formation and/or one or more methyl groups and/or oxygen atoms and/or by at least one mutation. At least one of modifications and mutations can be located in the core region.

The expression of the human epidermal growth factor receptor 2 is determined as up-regulated if amounts of CRIP1 and/or CRIP1 variants are increased compared to the control amounts, especially in the case of breast tissue.

The HER2 oncogene encodes a 185 ku epidermal growth factor receptor-related transmembrane protein. Due to this high molecular weight, it is important to note that mass spectrometry and especially MALDI IMS are not suited to directly detect HER2 as the typical observation mass range of this technology is 2.4 to 25 ku. The HER2 can not be found in body fluids due to the activity of proteases. According to the invention, protein profiles of HER2 "surrogate markers" are detected. These marker proteins are either co-expressed proteins with low molecular weights, fragments of HER2 or other high molecular weight proteins, CRIP1 variants and other proteins from the HER2 signaling pathway. In contrast to gene transcription experiments, it is preferred to measure the amounts of CRIP1 and at least one CRIP1 variant or the amounts of more than one CRIP1 variant to achieve results with adequate confidence levels.

In general the sample of the subject can be a body fluid, like whole blood, plasma, serum, nipple aspirate fluid, cerebrospinal fluid or urine, a homogenized or extracted tissue, a lysate of a homogenized sample or a tissue section. In the latter case the amounts of the marker proteins can be directly measured by imaging mass spectrometry, preferably utilizing matrix assisted laser desorption/ionization. The amounts of marker proteins, CRIP1 and/or one or more of CRIP1 variants in a sample can be measured by mass spectrometric means and/or by tandem mass spectrometry, e.g. by multiple reaction monitoring (MRM) following state of the art procedures. The tandem mass spectrometry further enables the identification of the measured proteins resulting in high accuracies of the methods. In addition, the amounts of CRIP1 and/or one or more of CRIP1 variants can be measured by an immunoassay (immunohisto-chemical analysis). Furthermore, the CRIP1 and/or one or more of CRIP1 variants can be extracted from the sample utilizing an antibody or other affinity reagents, like aptamers or affibodies, specific for CRIP1 or its variants. Hao et al. reported the use of phage display in combination with molecular modeling to identify a high-affinity ligand for CRIP1 (PLOS Computational Biology, 2008, 4(8), e1000138: "Identification and Rational Redesign of Peptide Ligands to CRIP1, A Novel Biomarker for Cancers"). The affinity extraction combined with mass spectrometric analysis or tandem analysis is a preferred embodiment. However, the measurement by IMS has the advantage that neither time consuming preparation steps nor expensive labeling or affinity reagents are needed.

The above methods for determining the expression of the human epidermal growth factor receptor 2 (HER2) of a subject can be used in different ways:
- for diagnosis of breast cancer, ovarian cancer, stomach cancer, uterine cancer, endometrial carcinoma, prostate cancer, cervical cancer or pancreatic cancer, wherein cancer is determined in case of an associated up-regulated expression of the human epidermal growth factor receptor 2 compared to healthy subjects,
- for qualifying of breast cancer, ovarian cancer, stomach cancer, uterine cancer, endometrial carcinoma, prostate cancer, cervical cancer or pancreatic cancer by the determined expression status of the human epidermal growth factor receptor 2,
- for predicting the response to a cancer drug, wherein a beneficial effect of drug is predicted in case of an up-regulated or down-regulated expression of the human epidermal growth factor receptor 2, or
- for predicting the response to a treatment with Trastuzumab against cancer, especially breast cancer, wherein a beneficial effect of Trastuzumab is predicted in case of an up-regulated expression of the human epidermal growth factor receptor 2.

>

### Description of the Drawings

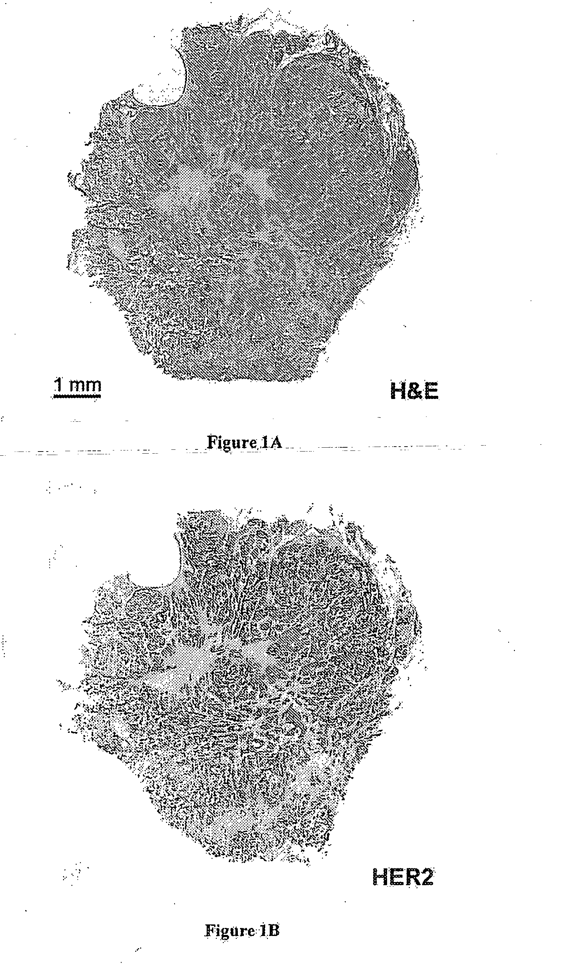

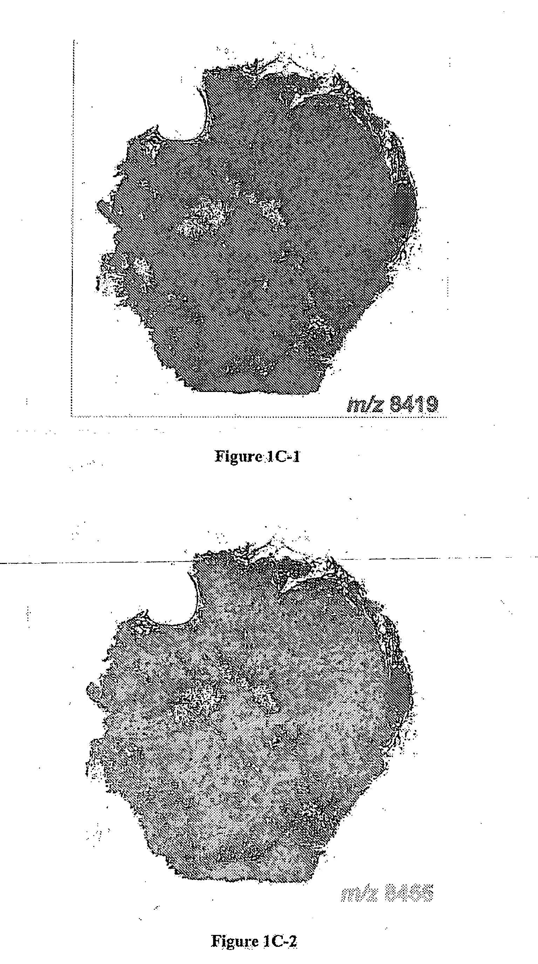

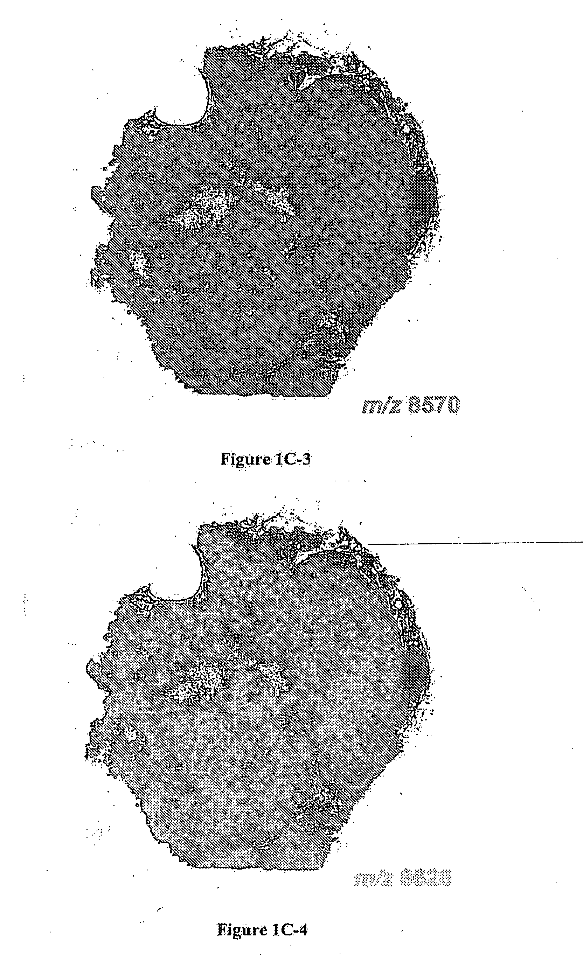

**Figure 1** MALDI IMS of a HER2-positive breast cancer tissue section. **A** Optical microscopic image of the hematoxylin and cousin (H&E) stained tissue section. The staining was done following the MALDI measurement of the very same tissue section. **B** Immunohistochemistry for HER² in a serial section of the breast cancer sample, which is classified as score 3+. **C-1 to C-4** Overlay of aligned H&E (A) and MALDI images. Four representative m/z species which are only present in HER2-positive tumors are selected for visualization of ion density images (m/z 8419, m/z 8455, m/z 8570, m/z 8626). These features are tumor cell specific and are not present in other cell types (e.g. stromal tissue). The Scale bar is 1mm.

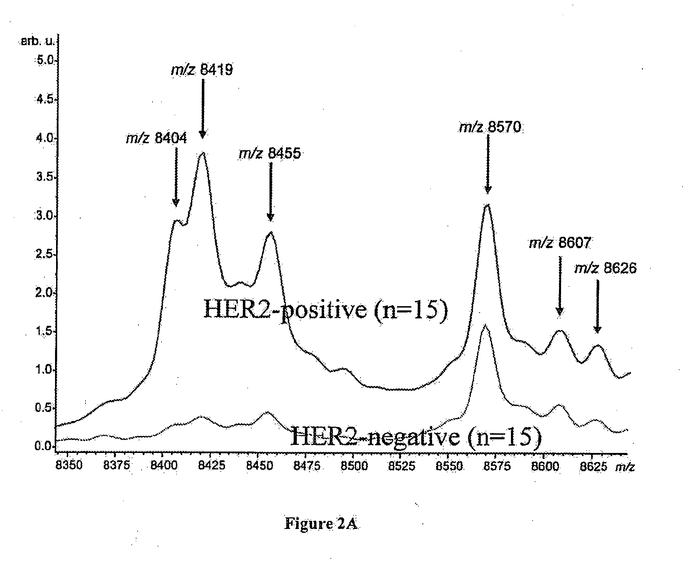

**Figure 2A** Examples of averaged MALDI-TOF MS spectra of HER2-positive (n=15) and HER2-negative (n=15) breast cancer tissues (discovery set) in the mass range of *m*/*z* 8345 to 8640. The arrows mark six peaks with significant differences between the HER2-positive and HER2-negative groups with centroid masses at *m*/*z* 8404, 8419, 8455, 8570, 8607, and 8626.

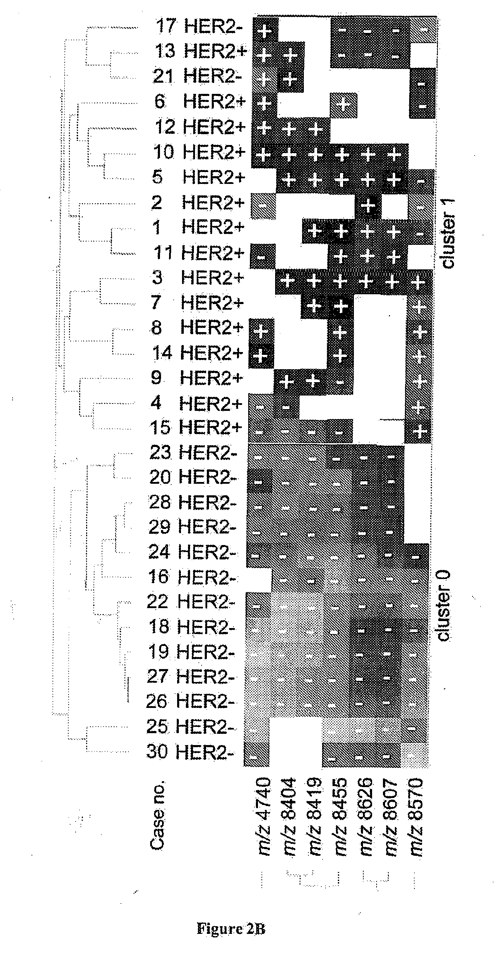

**Figure 2B** Hierarchical clustering of breast cancer tumor tissue specimens from the discovery set. All samples were clustered according to their expression pattern of the seven discriminating peptide ions at *m*/*z* 4740, 8404, 8419, 8455, 8570, 8607, and 8626. Peak intensities were standardized before clustering. Over-expressed peptide signals are shown as plus signs, under-expressed as minus signs and unchanged ones blank. The algorithm identified two clusters (shown as cluster 0 and cluster 1). While cluster 0 harbors only HER2-negative samples (n=13), two of the HER2-negative cases (17 and 21) were assigned as false positives to cluster 1 (HER2-positive group).

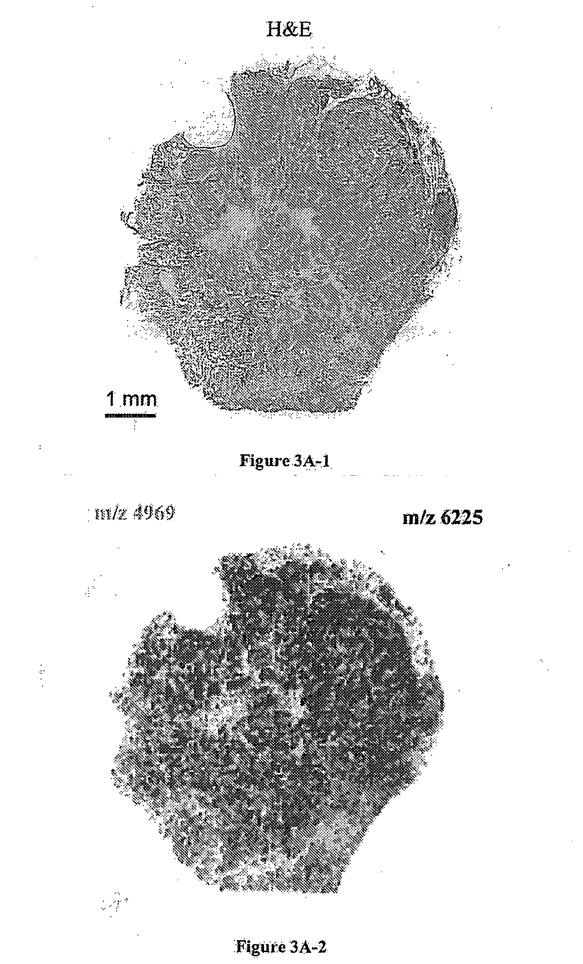

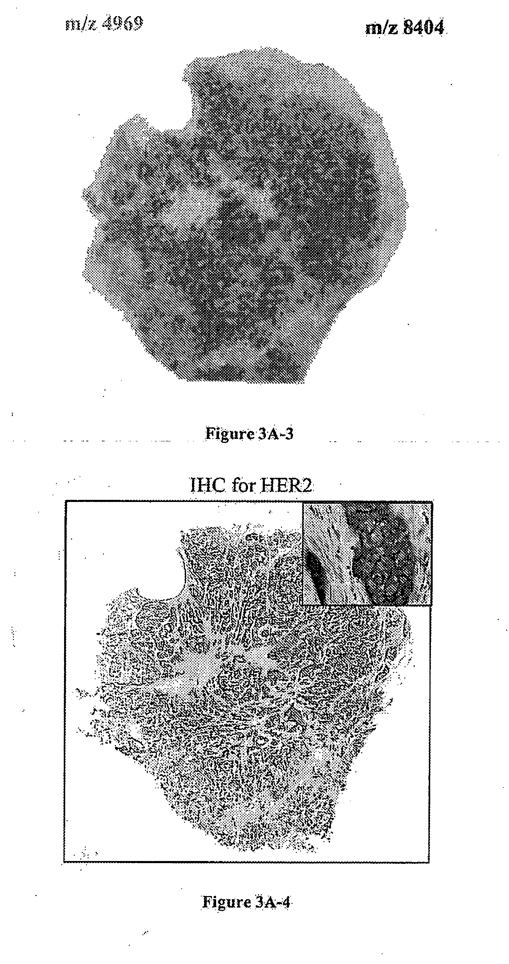

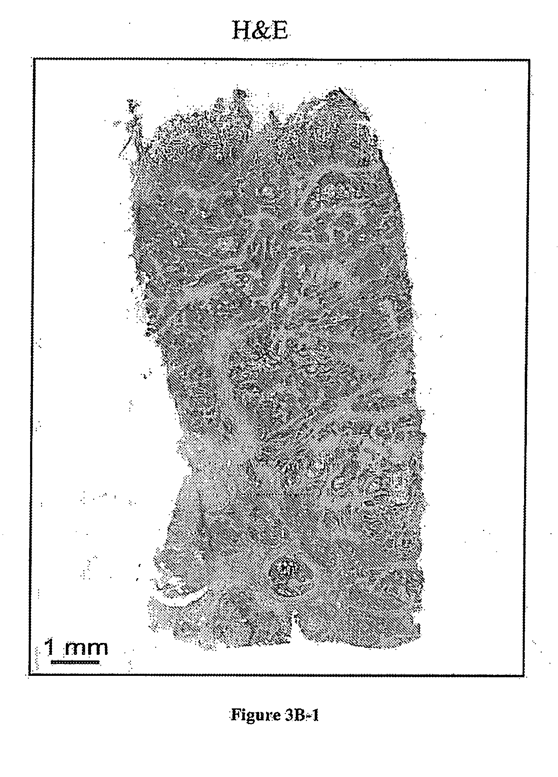

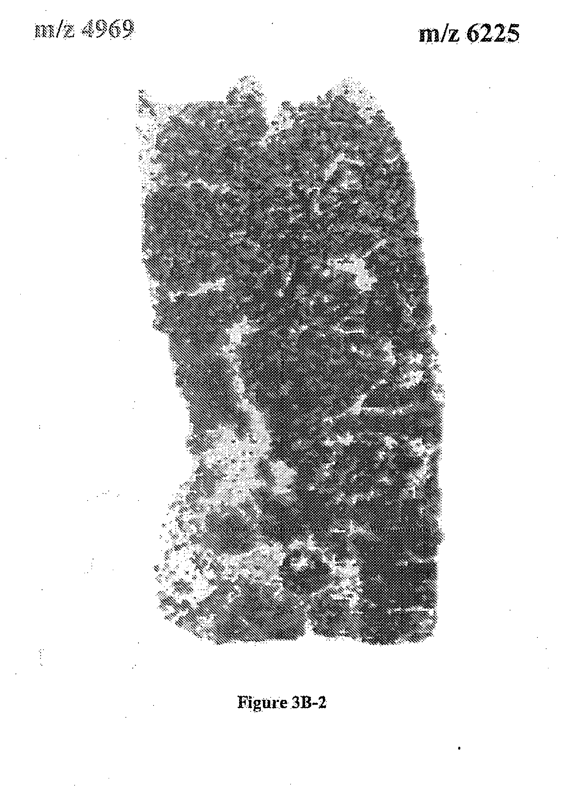

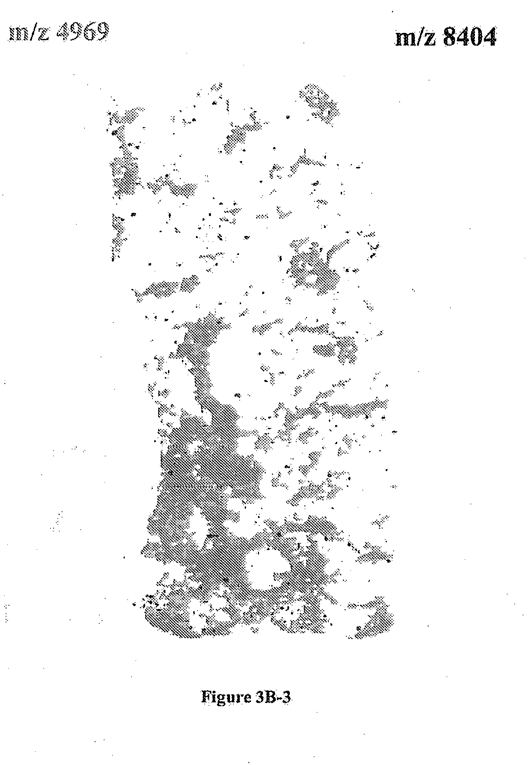

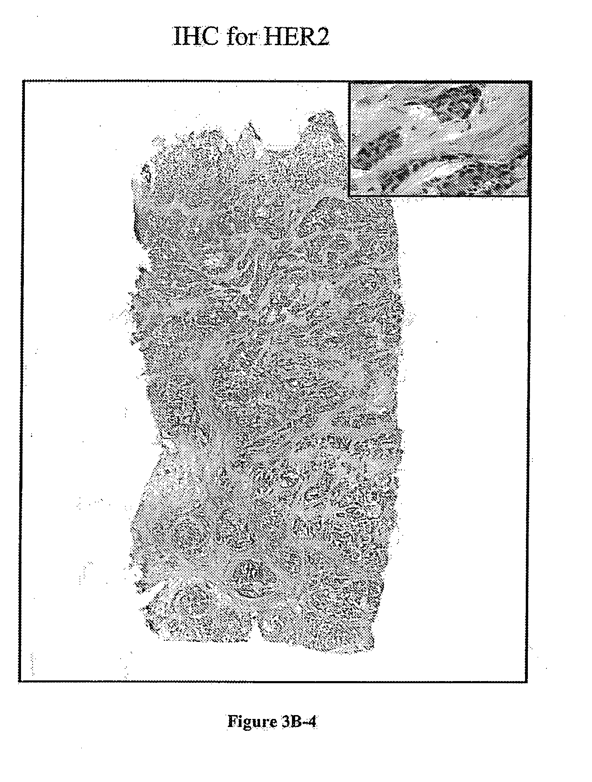

**Figure 3** Visualization of *m*/*z* 8404 as an example of a highly up-regulated spectral feature in HER2-positive breast cancer tissues: Optical microscopic images of the MALDI IMS measured and subsequently H&E stained original tissue sections of a HER2-positive case (Fig. 3A-1) and a HER2-negative case (Fig. 3B-1). Immunohistochemistry for HER2 of the respective case on a consecutive serial section are shown in Fig. 3A-4 and Fig. 3B-4, respectively. Note the strong immunoreactivity (score 3+) of the positive case while the reaction in the negative case indicates a score 0. The visualization of the *m*/*z* 8404 (Fig. 3A-3, Fig. 3B-3) found by MALDI IMS as highly up-regulated in HER2-positive tissues (Fig. 3A-3) clearly shows that this spectral feature is specific for cancer cells in this HER2-positive case but absent in the HER2-negative case (Fig. 3B-3). The spectral feature of *m*/*z* 6225 (Fig. 3A-2, Fig. 3B-2) is an example specific for cancer cells but not distinguishing between HER2-positive and HER2-negative tissues and is therefore present in both cases. *m*/*z* 4969 is a *m*/*z* species specific for tumor stroma and thus also present in both cases.

### Preferred Embodiment

The preferred embodiment describes the MALDI IMS technology for determining the HER2 expression status in breast cancer tissues, Clinical laboratory testing for HER2 expression status in newly diagnosed, primary breast cancer tissues is critically important for therapeutic decision making. Matrix-assisted laser desorption/iomzation (MALDI) imaging mass spectrometry (IMS) is a powerful tool for investigating proteins through the direct analysis of tissue sections. Unlike immunobistochemistry (IHC), MALDI-IMS enables the acquisition of complex protein expression profiles without any labeling.

Sample collection and tumor tissue specimens: Primary breast cancer patients presenting with invasive ductal carcinoma underwent primary surgical resection at the Department of Obstetrics and Gynecology, Klinikum rechts der Isar, Technische Universitat Munchen. Tumor tissue samples were snap-frozen and stored in liquid nitrogen in the tissue bank of the Institute of Pathology, Technische Universität Munchen. All tumor tissue specimens were procured from patients giving written informed consent. The study was approved by Ethics Committee of the Technische Universitat München. A total of 48 patients (30 for discovery set and 18 for validation set) were recruited for this study. The histomorphological and clinical characteristics of the analyzed cases are listed in Table 1.

Assessment of HER2 expression status: All cases were carefully reviewed before study inclusion for HER2 expression status. The discovery set was evaluated by imnunohistochemistry (IHC) and fluorescence in situ hybridization (FISH), while the validation set was evaluated by IHC only. Scoring criteria were applied according to the American Society of Clinical Oncology/College of American Pathologists guideline recommendations for HER2 testing in breast cancer (Wolff et al., 2007). A positive HER2 result is IHC staining of 3+ (uniform, intense membrane staining of> 30% of invasive tumor cells), a FISH result of more than six HER2 gene copies per nucleus or a FISH ratio (HER2 gene signals to chromosome 17 signals) of more than 2.2; a negative result is an IHC staining of 0 or 1+, a FISH result of less than 4.0 HER2 gene copies per nucleus or FISH ratio of less than 1.8. In total, 48 frozen tissue samples of invasive ductal breast cancer patients were used and the pre-characterized HER2 expression status was confirmed again by IHC on a consecutive cryosection.

MALDI Imaging Mass Spectrometry (MALDI IMS) experiments: Samples were cryosectioned on conductive Indium-Tin-Oxide (ITO) coated glass slides (Bruker Daltonik GmbH, Bremen, Germany), briefly washed in 70% and 100% ethanol, dried under vacuum, and directly covered with matrix. The MALI matrix was applied by use of the ImagePrep station (broker Daltonik GmbH, Bremen, Germany) and the standard protocol provided with the instrument, whereas sinapinic acid (Sigma-Aldrich, Taufkirchen, Germany) at 10 mg/ml in water/acetonitrile 40:60 (v/v) with 0.2% trifluoroacetic acid (TFA, Applied Biosystems, Darmstadt, Germany) was used as matrix for the MALDI measurements. The MALDI measurement was performed on an Ultraflex III MALDI-TOF/TOF in linear mode with a mass range of 2400-25000 u with a sampling rate of 0.1 GS/s using FlexControl 3.0 and FlexImaging 2.1 software packages (all Bruker Daltonik GmbH, Bremen, German). The lateral resolution for MALDI IMS was set to 200 µm and a total of 200 laser shots were accumulated per pixel at constant laser power.

Following the MALDI IMS experiments, the cryosections were incubated in 70% ethanol to remove the matrix and then washed in distilled water. Subsequently, the very same section, which was measured by MALDI IMS, was stained with hematoxylin and eosin (H&E), scanned with the MIRAX^{®} DESK system (Carl Zeiss Micro Imaging GmbH, Göttingen, Germany), and co-registered with the MALDI IMS results to correlate mass spectrometric data with the histological features of the section.

Statistical analyses: Tumor associated spectra were selected using the FlexImaging 2.1 software (Bruker). For this purpose, we defined in every tissue section regions of interest (ROIs) that contained invasive ductal breast cancer tissue specific for HER2-positive and HER2-negative cell populations, respectively. These extracted mass spectra comprising about 300 peaks per spectrum underwent recalibration on common "background" peaks (also known as spectral alignment) and normalization based on their total ion count in the observation mass range utilizing ClinPro Tools 2.2 software (Bruker Daltonik GmbH, Bremen, Germany). An average spectrum across all spectra of an ROI was used for peak defining integration ranges. These integration ranges were used to obtain the average peak intensities of each respective ROI. Significant differences in peak intensities between both histological groups (HER2-positive and HER2-negative) were evaluated by the wilco on rank-sum test with a significance cutoff point of p < 0.05.

For classification and further statistical analyses, data were exported to the R statistical software (R Foundation for Statistical Computing) in order to compare the quality of two different classification algorithms predicting HER2 expression status. For this purpose, a support vector machine (SVM) and an artificial neuronal network (ANN) were computed on the discovery set of samples. Both classifiers were tested internally and externally using a 10-fold cross validation and the validation set, respectively. At each evaluation step the features were selected that best segregated the samples within the training set into the related groups (i.e. HER2-positive or HER2-negitive), where an MS signal was included once its area under curve (AUC) exceeded 0.8 as calculated by receiver operating characteristics (ROC) analysis. Finally, the performances of both approaches were compared by analyzing the resulting confusion matrices across all evaluation steps.

The additional hierarchical clustering of the expression profiles of all samples was carried out with the software PLUTO (George Karypis, Department of Computer Science & Engineering, University of Minnesota, USA) on the standardized, exported data. CLUTO is a software package for clustering low- and high-dimensional datasets and for analyzing the characteristics of the various clusters.

Results: In the initial discovery experiment, we analyzed 30 tumor tissue samples (15 HER2-negative samples, 15 HER2-positive samples). 1129 individual mass spectra, representing specific HER2-positive or HER2-negative tumor cell populations defined by ROIs, were used for comparative analyses. Representative example of images derived from mapping discriminating peaks are shown in Figures 1 and 3. Another peak at *m*/*z* 4969 was expressed in tumor stroma with little or no expression seen in cancer cells (Figures 3A-2, 3A-3, 3B-2 and 3B-3). In the mass range *m*/*z* 8345 to 8640, five peptide ions could be detected, which showed an over-expression in the HER2-positive cases.compared to the HER2-negative cases (Figures 1C-1 to 1C-4 and 2A). In total, seven *m*/*z* species were found at an average of *m*/*z* 4740, *m*/*z* 8404, *m*/*z* 8419, *m*/*z* 8455, *m*/*z* 8570, *m*/*z* 8607 and *m*/*z* 8626, which discriminate between the HER2-positive and the HER2-negative tumor samples. These peaks were clearly different from the background noise and were determined with a mass accuracy of ±4 u average molecular weight in linear mode. Besides the above MS signals, there is another differentiating MS signal (not shown in the Figures 1 to 3) at about *m*/*z* 22490 being specific for invasive ductal cancer cells but not present in tumor stroma or other tissue components.

According to the expression pattern of the above described seven *m*/*z* species, a hierarchical clustering was performed on the samples of the discovery set (Figure 2B). The algorithm identified two clusters. While one cluster harbors HER2-negative samples (n=13), only two HER2-negative samples (case numbers 17 and 21) were assigned as false-positives to the HER2-positive designated group.

For performance assessment of both classifiers (SVM and ANN) first a 10-fold cross-validation was applied on the sample data (discovery set). The ANN outperformed the SVM by gaining a correct classification rate of 90% allowing a reliable discrimination of the HER2-positive and HER2-negative groups. The classification ability of both classifiers (SVM and ANN) was furthermore used to predict the HER2 expression status of the tissues from the validation set (n=8). This resulted in a misclassification of two cases. Therefore both approaches achieved an overall accuracy of 89%, with a Sensitivity of 83% and a specificity of 92% (Table 1).

Identification of one of the marker proteins by tissue lysis and tandem mass spectrometric analysis: Two human HER2-positive breast cancer biopsy slices (10 µm thickness, not previously stained or analyzed by MALDI imaging) were placed in an 1.5 ml Eppendorf tube with 50 µl 0.1% TFA followed by a 2 min centrifugation (20,000 g) to spin down the tissue (Eppendorf Centrifuge 5804 R, Eppendorf, Germany). To lyse the tissue, ultrasonication in an ice bath was applied for 30 min. Subsequently, centrifugation (20,000 g) was performed at 4°C for 30 min. Tissue lysate was further processed with ultrafiltration (Vivaspin 500, cutoff 5 ku, Sartorius Stedim Biotech, France). 25 µl of supernatant from centrifugation was pipette into a spin column and centrifuged for one hour at 15,000 g. To wash the lysate, 25 µl 0.1%TFA was added to the spin column and centrifuged again for one hour. Spin column was refilled to 25 µl (0.1% TFA), the membrane was rinsed with this solution to obtain proteins and the solution filled into an auto sampler vial. Intact proteins were then separated with an Agilent mRP column (5 µm, 0.5 x 100 mm) and Agilent 1200 HPLC system (Agilent Technologies, Böblingen, Germany). Following LC parameters were used: Flow rate 12 µl/min, column oven set to 60°C, injection volume 8 µl, solvent A 0.1% formic acid (Sigma-Aldrich) in water, solvent B 0.1% formic acid in acetonitrile (ACN, Sigma-Aldrich), step wise gradient 7 min at 3% B, in 23 min to 22% B, in 5 min to 38% B, in 2 min to 97% B, 5 min at 97% B, ion 2 min to 3% B, and 5 min at 3% B. Fractionation was performed with a Proteineer fc (Bruker Daltonik GmbH, Bremen, Germany) from 10-42 main with 20 sec time slices into a 96 well plate; two separations were collected in one well plate. For candidate localization 0.5 µl from collected fractions were spotted onto 96PAC target and analyzed with an Ultraflex III MALDI-TOF/TOF in linear mode (both: Bruker Daltonik GmbH, Bremen, Germany).

For ETD/PTR fragmentation three subsequent fractions containing the candidate peptide were combined (approx. 15 µl) and diluted with 1.5 µl 2-propanol (Sigma-Aldrich) and 7.5 µl ACN/ H₂0 with 0.2% formic acid. Infusion MS experiments were performed with TriVersa NanoMate (10 µl sample, 384 well plate, D-chip width 0.4 psi and 1.55 kV; Advion Biosystems, Ithaca, NY, USA) and amaZon ETD Ion Trap (Bruker Daltonik GmbH, Bremen, Germany) as described previously (Hartmer et al., 2008). Briefly, the sample was measured with Maximum Scan Mode at 4600/sec, polarity positive, Scan Range from *m*/*z* 100-3000, 10 spectra averaged and rolling average of 4. Chosen precursor was *m*/*z* 602.4 (charge state 14⁺) with analyte ICC of 125,000 and isolation width of *m*/*z* 4.0, MS² stage was ETD with reaction time of 45 ms and reactant ICC of 115,000, MS³ stage was PTR with reaction time of 80 ms and reactant ICC of 175,000. Acquired ETD/PTR spectra were processed using DataAnalysis 4.0 with the SNAPII algorithm, deconvoluted spectra were further analyzed with BioTools 3.2 (all Bruker Daltonik GmbH, Bremen, Germany) and submitted to a Mascot (Matrix Science, London, UK) database search (Mascot 2.2.04, SwissProt 56.1, taxonomy: mammals, Peptide mass tolerance +/- 10 u, fragment mass tolerance +/- 0.8 u, Instrument type ETD-Trap). The identified protein sequence was manually validated in BioTools on a residue-by residue basis using the raw data.

Cystein-rich protein 1 (CRIP1_HUMAN) was identified by database searching with a mascot score of 126. A theoretical average molecular weight of 8402 u was calculated from the underivatized CRIP1_HUMAN des-Met 1 gene sequence in good agreement with the MALDI and ESI data. However, a cluster of CRIP1 variants at 8419, 8435, 8450 and 8464 u were determined experimentally after charge deconvolution of the multiple charge states in the ion trap ESI spectrum. The varying molecular weights result from one or more structural variations, wherein the human cystein-rich intestinal protein 1 is modified, e.g., by disulfide bond formation and/or one or more methyl groups and/or oxygen atoms and/or by at least one mutation.

The MALDI IMS proteomic approach, as one preferred embodiment described above, is a label-free and morphology-driven alternative for HER2 testing in breast cancer tissues and will have great implications for clinical management of breast cancer patients, and may be applicable to other cancer diseases as well. Only small amounts of fresh-frozen and unprocessed tumor tissue, easily available in a clinical setting, are needed to obtain the profiles of the marker proteins that can be used to accurately classify tumors associated with molecular features such as HER2. The tissue sample can be gained from core needle biopsies or can be available as a tissue microarray set.

### References

Cornett DS, Reyzer ML, Chaurand P, Caprioli RM: MALDI imaging mass spectrometry: molecular snapshots of biochemical systems. Nat Methods. 2007;4(10):828-33.
Franck J, Arafah K, Elayed M, Bonnel D, Vergara D, Jacquet A, Vinatier D, Wisztorski M, Day R, Fournier 1, Salzet M: MALDI IMAGING: State of the art technology in clinical proteomics. Mol Cell Proteomics. 2009 May 18
Liu S, Stromberg A, Tai H, Moscow JA: Thiamine transporter gene expression and exogenous thiamine modulate the expression of genes involved in drug and prostaglandin metabolism in breast cancer cells, Mol. Cancer Res. 2004;2:477-487.
Ma XJ, Salunga R, Tuggle JT, Gaudet J, McQuary P, et al: Gene expression profiles of human breast cancer progression. Proc Natl Acad Sci U S A. 2003;100:1974-5979.
Mackay A, Jones C, Dexter T, Silva RL, Bulmer K, Jones A, Simpson P, Harris RA, Jat PS, Neville AM, Reis LF, Lakhani SR, O'Hare MJ: cDNA microarray analysis of genes associated with ERBB2 (HER2/neu) overexpression in human mammary luminal epithelial cells. Oncogene. 2003;22(17):2680-8.
Schwartz SA, Weil RJ, Thompson RC, Shyr Y, Moore JH, Toms SA, Johnson MD. Caprioli RM: Proteomic-based prognosis of brain tumor patients using direct-tissue matrix-assisted laser desorption ionization mass spectrometry, Cancer Res. 2005 Sep 1;65(17):7674-81.
Wilson KS, Roberts H, Leek R, Harris AL, Geradts J: Differential gene expression patterns in HER2/neu-positive and -negative breast cancer cell lines and tissues. Am J Pathol, 2002;161(4):1171-85.
Yanagisawa K, Shyr Y, Xu BJ, Massion PP, Larsen PH, White BC, Roberts JR, Edgerton M, Gonzalez A, Nadaf S, Moore JH, Caprioli RM, Carbone DP: Proteomic patterns of tumour subsets in non-small-cell lung cancer, The Lancet, Volume 362, Issue 9382, 9 August 2003, Pages 433-439

**Table 1**

| Clinical and molecular characteristics of the patient series | | | |
|---|---|---|---|
| Set characteristics | | Discovery set | Validation set |
| Patients | | 30 | 18 |
| Mean age (range) | | 60.1 (38-91) | 61.4 (36-84) |
| HER2 expression status | | | |
| IHC | | | |
| | Score 3+ | 13 | 6 |
| | Score 2+ | 2 | 0 |
| | Score 1+ | 2 | 6 |
| | Score 0 | 13 | 6 |
| FISH | | | |
| | positive | 15 | n.a. |
| | negative | 15 | n.a. |
| pT classification | | | |
| pT1mic | | 0 | 1 |
| pT1a | | 2 | 0 |
| pT1b | | 1 | 0 |
| pT1c | | 9 | 0 |
| pT2 | | 16 | 11 |
| pT3 | | 1 | 1 |
| pT4 | | 1 | 0 |
| pTx | | 0 | 5 |
| pN classification | | | |
| positive | | 17 | 6 |
| negative | | 12 | 7 |
| pNx | | 1 | 5 |
| Grading | | | |
| Grade 1 | | 0 | 0 |
| Grade 2 | | 3 | 6 |
| Grade 3 | | 27 | 12 |
| ER / PR status | | | |
| ER+ | | 14 | 5 |
| ER- | | 16 | 13 |
| PR+ | | 9 | 13 |
| PR- | | 21 | 5 |
| | | | |

| Proteomic classification | | Discovery set* | Validation set |
|---|---|---|---|
| Support Vector Machine | | | |
| Accuracy | | 87% | 89% |
| | | (95% CI 70% - 96%) | (95% CI 65% - 99%) |
| Sensitivity | | 87% | 83% |
| Specificity | | 87% | 92% |
| | | | |

| Artificial Neuronal Network | | | |
|---|---|---|---|
| Accuracy | | 90% | 89% |
| | | (95% CI 73% - 98%) | (95% CI 65% - 99%) |
| Sensitivity | | 87% | 83% |
| Specificity | | 93% | 92% |
| | | | |

Abbreviations used in Table 1:
HER2 = human epidermal growth factor receptor 2,
IHC = immunohistochemistry,
FISH = fluorescence *in situ* hybridization,
ER = estrogen receptor,
PR = progesterone receptor,
CI = confidence interval,
n,a. = not analyzed
* Classifiers were evaluated using a 10-fold cross-validation

## Claims

1. A method for determining the expression of the human epidermal growth factor receptor 2 (HER2) of a subject in order to determine the most appropriate breast cancer treatment, comprising:
(a) measuring amounts of two proteins in a sample of the subject, wherein the two proteins have a molecular weight of 8404 atomic mass units and 8419 atomic mass units, said molecular weights being assigned to the human cystein-rich intestinal protein 1 (CRIP1) or a structurally modified variant human cystein-rich intestinal protein 1, respectively
and
(b) comparing the amounts of the proteins to control amounts determinative of the expression of the human epidermal growth factor receptor 2, wherein the expression of the human epidermal growth factor receptor 2 is determined as up-regulated if the amounts of the two proteins compared to the control amounts is increased.

2. The method of claim 1, wherein the amount of at least one further protein is measured and compared to a control amount, the at least one further protein having a molecular weight of 4740, 8455, 8570, 8607 or 8626 atomic mass units and being related to fragments of the human epidermal growth factor receptor 2.

3. The method of claim 1, wherein the amount of at least one further protein is measured and compared to a control amount, the at least one further protein having a molecular weight of 8435, 8450 or 8465 atomic mass units and being related to variants of the human cystein-rich intestinal protein 1.

4. The method of claim 1, wherein the structural variation is at least one of an in vivo or in vitro modification, a post-translational modification, a sequence variation, and a point mutation.

5. The method of claim 1, wherein the amounts of the two proteins are measured either by an immunoassay or by mass spectrometry, or is measured and optionally identified by tandem mass spectrometry.

6. The method of claim 1, wherein the human cystein-rich intestinal protein 1 either is modified by one or more structural variations, such as at least one of disulfide bond formation, one or more methyl groups, and oxygen atoms, or is modified by at least one mutation.

7. The method of claim 6, wherein at least one of modifications and mutations is located in the core region.

8. The method of claim 1, wherein the sample of the subject is either one of a body fluid, such as whole blood, plasma, serum, nipple aspirate fluid, cerebrospinal fluid or urine, or homogenized or extracted tissue, such as a lysate of a homogenized tissue, or a tissue section.

9. The method of claim 8, wherein the tissue section is analyzed by imaging mass spectrometry, such as imaging mass spectrometry utilizing matrix assisted laser desorption/ionization.

10. The method of claim 1, wherein breast cancer, ovarian cancer, stomach cancer, uterine cancer, endometrial carcinoma, prostate cancer, cervical cancer or pancreatic cancer either is determined in case of an up-regulated expression of the human epidermal growth factor receptor 2 compared to healthy subjects, or is qualified by the determined expression status of the human epidermal growth factor receptor 2.

11. The method of claim 1, wherein a beneficial effect of a drug is predicted in case of an up- or down-regulated expression of the human epidermal growth factor receptor 2.

12. The method of claim 11, wherein a beneficial effect of Trastuzumab is predicted in case of an up-regulated expression of the human epidermal growth factor receptor 2.

13. The method of claim 1, wherein the proteins are extracted from the sample utilizing an antibody or other affinity reagents, like aptamers or antibodies, specific for CRIP1 or its variants and wherein the amounts of the two proteins are measured by mass spectrometry or by tandem mass spectrometry.

14. The method of claim 9, wherein the tissue section is a breast cancer tissue section.

## Patentansprüche

1. Verfahren zum Bestimmen der Expression des menschlichen epidermalen Wachstumsfaktor-Rezeptors 2 (HER2) eines Subjektes zum Bestimmen der am Besten geeigneten Brustkrebsbehandlung, umfassend:
(a) Messen der Mengen von zwei Proteinen in einer Probe des Subjekts, wobei die beiden Proteine ein Molekulargewicht von 8404 atomaren Masseneinheiten und 8419 atomaren Masseneinheiten haben, wobei die genannten Molekulargewichte dem menschlichen Cystein-reichen Darmprotein 1 (CRIP1) bzw. einer strukturell modifizierten Variante des menschlichen Cystein-reichen Darmproteins 1 zugeordnet sind und
(b) Vergleichen der Mengen des Proteins mit Kontrollmengen, die für die Expression des menschlichen epidermalen Wachstumsfaktor-Rezeptors 2 entscheidend sind, wobei die Expression des menschlichen epidermalen Wachstumsfaktor-Rezeptors 2 als hochreguliert bestimmt wird, falls die Mengen der zwei Proteine im Vergleich zu den Kontrollmengen erhöht sind.

2. Verfahren nach Anspruch 1, wobei die Menge mindestens eines weiteren Proteins gemessen und mit einer Kontrollmenge verglichen wird, wobei das mindestens eine weitere Protein ein Molekulargewicht von 4740, 8455, 8570, 8607 oder 8626 atomaren Masseneinheiten hat und sich auf Fragmente des menschlichen epidermalen Wachstumsfaktor-Rezeptors 2 bezieht.

3. Verfahren nach Anspruch 1, wobei die Menge mindestens eines weiteren Proteins gemessen und mit einer Kontrollmenge verglichen wird, wobei das mindestens eine weitere Protein ein Molekulargewicht von 8435, 8450 oder 8465 atomaren Masseneinheiten hat und sich auf Varianten des menschlichen Cystein-reichen Darmproteins 1 bezieht.

4. Verfahren nach Anspruch 1, wobei die strukturelle Variation mindestens eine der Folgenden ist: in vivo oder in vitro Modifikation, posttranslationale Modifikation, Sequenz-Variation oder Punktmutation.

5. Verfahren nach Anspruch 1, wobei die Mengen der zwei Proteine entweder durch ein Immunoassay oder durch Massenspektrometrie gemessen werden oder gemessen und optional mittels Tandem-Massenspektrometrie identifiziert werden.

6. Verfahren nach Anspruch 1, wobei das menschliche Cystein-reiche Darmprotein 1 entweder durch eine oder mehrere strukturelle Variationen modifiziert wird, wie beispielsweise mindestens eine der Folgenden: Disulfidbindungs-Bildung, eine oder mehrere Methylgruppen, und Sauerstoffatome, oder durch mindestens eine Mutation modifiziert wird.

7. Verfahren nach Anspruch 6, wobei mindestens eine der Modifikationen und Mutationen in dem Kernbereich liegt.

8. Verfahren nach Anspruch 1, wobei die Probe des Subjekts entweder eine Körperflüssigkeit wie Vollblut, Plasma, Serum, Nippelaspiratflüssigkeit, Cerebrospinalflüssigkeit oder Urin, oder homogenisiertes oder extrahiertes Gewebe, wie Lysat eines homogenisierten Gewebes, oder ein Gewebeschnitt ist.

9. Verfahren nach Anspruch 8, wobei der Gewebeschnitt durch Bildgebende Massenspektrometrie, wie beispielsweise Bildgebende Massenspektrometrie unter Verwendung von Matrix-unterstützter Laserdesorption/Laserionisation analysiert wird.

10. Verfahren nach Anspruch 1, wobei Brustkrebs, Eierstockkrebs, Magenkrebs, Gebärmutterkrebs, Endometriumkarzinome, Prostatakrebs, Gebärmutterhalskrebs, oder Bauchspeicheldrüsenkrebs entweder festgestellt wird im Falle einer hochregulierten Expression des menschlichen epidermalen Wachstumsfaktor-Rezeptors 2 im Vergleich mit gesunden Subjekten oder durch den bestimmten Expressionsstatus des menschlichen epidermalen Wachstumsfaktor-Rezeptors 2 in Frage kommt.

11. Verfahren nach Anspruch 1, wobei eine positive Auswirkung eines Medikaments im Falle einer hoch- oder herunterregulierten Expression des menschlichen epidermalen Wachstumsfaktor-Rezeptors 2 vorausgesagt wird.

12. Verfahren nach Anspruch 11, wobei eine positive Auswirkung von Trastuzumab im Falle einer hochregulierten Expression des menschlichen epidermalen Wachstumsfaktor-Rezeptors 2 vorausgesagt wird.

13. Verfahren nach Anspruch 1, wobei die Proteine aus der Probe extrahiert werden unter Verwendung eines Antikörpers oder anderer Affinitätsreagenzien, wie Aptamere oder Antikörper, die für CRIP1 oder seine Varianten spezifisch sind, und wobei die Mengen der zwei Proteine durch Massenspektrometrie oder Tandem-Massenspektrometrie gemessen werden.

14. Verfahren nach Anspruch 9, wobei der Gewebeschnitt ein Brustkrebs-Gewebeschnitt ist.

## Revendications

1. Procédé de détermination de l'expression du récepteur 2 du facteur de croissance épidermique humain (HER2) d'un sujet afin de déterminer le traitement du cancer du sein le plus approprié, comprenant les étapes consistant à :
(a) mesurer des quantités de deux protéines dans un échantillon du sujet, où les deux protéines présentent un poids moléculaire de 8404 unités de masse atomique et 8419 unités de masse atomique, lesdits poids moléculaires étant attribués à la protéine intestinale riche en cystéine 1 humaine (CRIP1) ou une variante de la protéine intestinale riche en cystéine 1 humaine modifiée structurellement, respectivement
et
(b) comparer les quantités des protéines à des quantités de contrôle déterminant l'expression du récepteur 2 du facteur de croissance épidermique humain, où l'expression du récepteur 2 du facteur de croissance épidermique humain est déterminée comme étant à régulation à la hausse si les quantités des deux protéines comparées à la quantité de contrôle ont augmenté.

2. Procédé selon la revendication 1, dans lequel la quantité d'au moins une autre protéine est mesurée et comparée à une quantité de contrôle, la au moins une autre protéine présentant un poids moléculaire de 4740, 8455, 8570, 8607 ou 8626 unités de masse atomique et étant liée à des fragments du récepteur 2 du facteur de croissance épidermique humain.

3. Procédé selon la revendication 1, dans lequel la quantité d'au moins une autre protéine est mesurée et comparée à une quantité de contrôle, la au moins une autre protéine présentant un poids moléculaire de 8435, 8450 ou 8465 unités de masse atomique et étant liée à des variantes de la protéine intestinale riche en cystéine 1 humaine.

4. Procédé selon la revendication 1, dans lequel la variation structurelle est au moins l'une d'une modification in vivo ou in vitro, d'une modification post-traduction, d'une variation de séquence et d'une mutation ponctuelle.

5. Procédé selon la revendication 1, dans lequel les quantités des deux protéines sont mesurées soit par un immuno-essai soit par spectrométrie de masse, ou bien est mesurée et optionnellement identifiée par une spectrométrie de masse en tandem.

6. Procédé selon la revendication 1, dans lequel la protéine intestinale riche en cystéine 1 humaine est soit modifiée par une ou plusieurs variations structurelles, telles qu'au moins une formation de liaison disulfure, un ou plusieurs groupes méthyle, et des atomes d'oxygène, soit est modifiée par au moins une mutation.

7. Procédé selon la revendication 6, dans lequel au moins l'une des modifications et des mutations est située dans la région centrale.

8. Procédé selon la revendication 1, dans lequel l'échantillon du sujet est soit l'un d'un fluide corporel, comme du sang complet, du plasma, du sérum, d'un fluide d'aspiration du mamelon, d'un fluide cérébrospinal ou de l'urine, soit un tissu homogénéisé ou extrait, comme un lysat d'un tissu homogénéisé, ou une section de tissu.

9. Procédé selon la revendication 8, dans lequel la section de tissu est analysée par une spectrométrie de masse d'imagerie, telle qu'une spectrométrie de masse d'imagerie utilisant une désorption/ionisation laser assistée par matrice.

10. Procédé selon la revendication 1, dans lequel un cancer du sein, un cancer de l'ovaire, un cancer de l'estomac, un cancer de l'utérus, un carcinome de l'endomètre, un cancer de la prostate, un cancer du col de l'utérus ou un cancer du pancréas soit est déterminé dans le cas d'une expression à régulation à la hausse du récepteur 2 du facteur de croissance épidermique humain comparée à des sujets sains, soit est qualifié par l'état d'expression déterminé du récepteur 2 du facteur de croissance épidermique humain.

11. Procédé selon la revendication 1, dans lequel un effet bénéfique d'un médicament est prédit dans le cas d'une expression à régulation à la hausse ou à la baisse du récepteur 2 du facteur de croissance épidermique humain.

12. Procédé selon la revendication 11, dans lequel un effet bénéfique du Trastuzumab est prédit dans le cas d'une expression à régulation à la hausse du récepteur 2 du facteur de croissance épidermique humain.

13. Procédé selon la revendication 1, dans lequel les protéines sont extraites de l'échantillon en utilisant un anticorps ou d'autres réactifs à affinité, comme des aptamères ou des anticorps, spécifiques à la protéine CRIP1 ou ses variantes et dans lequel les quantités des deux protéines sont mesurées par spectrométrie de masse ou par spectrométrie de masse en tandem.

14. Procédé selon la revendication 9, dans lequel la section de tissu est une section de tissu de cancer du sein.
